# EUROPÄISCHE PATENTSCHRIFT

(11) **EP 1 043 011 B1**
(45) Veröffentlichungstag und Bekanntmachung des Hinweises auf die Patenterteilung: **16.06.2004**
(21) Anmeldenummer: 00105303.2
(22) Anmeldetag: 15.03.2000
(51) Int. Cl.: A61K 7/06

(54) **Zweistufiges Verfahren zur Konditionierung von Haaren und zum Erhalten der Frisur**
Two-step hair conditioning and style retention process
Procédé de conditionnement du cheveu et du maintien de la coiffure en deux étapes

(30) Priorität: 09.04.1999 DE 19916027
(43) Veröffentlichungstag der Anmeldung: 11.10.2000
(73) Patentinhaber: KPSS-Kao Professional Salon Services GmbH, 64297 Darmstadt (DE)
(72) Erfinder: Dubowoj, Polina, 64319 Pfungstadt (DE)

(56) Entgegenhaltungen:
- EP-A- 0 640 643
- DE-A- 19 738 303
- DE-C- 19 751 589
- US-A- 5 726 137

## Beschreibung

Die vorliegende Erfindung betrifft ein Verfahren zur Haarbehandlung, insbesondere von frisch gewaschenem Haar, durch das dem Haar ein besonders weicher Griff, erhöhter Glanz, verbesserte Elastizität, Flexibilität und Sprungkraft und insbesondere eine gute Wiederfrisierbarkeit verliehen werden.

Mittel zum Konditionieren von menschlichen Haaren sind seit langem bekannt. Sie enthalten in der Regel quaternäre Ammoniumverbindungen, die mindestens eine langkettige Alkyl- oder Alkenylgruppe aufweisen, und gegebenenfalls auch Polymere.
Solche Mittel werden üblicherweise als wäßrige Dispersionen bzw. Emulsionen, Mikroemulsionen, Gele oder auch in Aerosolform konfektioniert und als Haarspülungen, Kuren etc. eingesetzt.
Eine Übersicht über die bekannten Haarnachbehandlungsmittel und ihre Zusammensetzung findet sich in der Monographie von K. Schrader, Grundlagen und Rezepturen der Kosmetika, 2. Aufl. (1989), S. 728 bis 737.

Ebenso werden seit langem auch haarfestigende Mittel, die in der Regel mindestens ein filmbildendes natürliches oder synthetisches Polymeres enthalten, in vielfältigen Applikationsformen wie Lösungen, Gelen und insbesondere Aerosolzusammensetzungen wie Haarsprays oder Schaumaerosolen eingesetzt.

Auch diese Zusammensetzungen sind im Prinzip in der Monographie von Schrader, S. 737-760 und 771-780 beschrieben.

Sowohl Nachbehandlungsmittel als auch Festiger wie Haarsprays und Styling-Präparate werden üblicherweise im Anschluß an eine Haarbehandlung, insbesondere eine Haarwäsche oder auch eine Färbung oder Dauerwellung mit abschließender Wäsche oder Spülung auf das Haar aufgebracht.

Aus der US 5,726,137 sind Shampoos bekannt, die sowohl quaternäre Ammoniumverbindungen als auch filmbildende Polymere enthalten, die die Haare waschen, konditionieren als auch stylen und somit zu den "3 in 1 Produkten" zählen.
Die DE 197 51 589 C1 beschreibt ein Haarbehandlungsmittel, insbesondere ein Haarkonditioniermittel, das dem menschlichen Haar Volumen, erhöhten Galnz und leichte Nass- und Trockenkämmbarkeit verleiht sowie auch in der Lage ist, geschädigtes Haar zu regenerieren.
Die EP 0 640 643 A2 zeigt ein Haarbehandlungsmittel auf, das Organopolysiloxan enthält und zur Festigung und Erhaltung der Frisur verwendet wird.
Aus der DE 197 38 303 A1 ist die Verwendung einer speziellen Wirkstoffkombination zum Schutz von keratinischen Fasern vor Schäden durch Hitzeeinwirkung sowie diese Wirkstoffkombination enthaltende Mittel bekannt.

Es wurde nun gefunden, und das ist Gegenstand der vorliegenden Erfindung, daß menschlichem Haar ein besonders weicher Griff, erhöhter Glanz, verbesserte Elastizität, Flexibilität und Sprungkraft sowie insbesondere eine gute Wiederfrisierbarkeit, d.h., Herstellung der ursprünglichen Frisur nach längerer Zeit, beispielsweise der Nachtruhe, durch einfaches Kämmen, ein sogenannter "Memory-Effekt" verliehen werden können, wenn man auf frisch gewaschenes Haar zunächst in an sich bekannter Weise eine Zusammensetzung I, enthaltend 0,25 bis 15 Gew.-%, berechnet auf die Zusammensetzung, mindestens einer haarkonditionierenden langkettigen quaternären Ammoniumverbindung, und/oder Aminverbindung, und anschließend, ohne zwischenzeitliches Ausspülen, eine Zusammensetzung II, enthaltend 0,25 bis 10 Gew.-%, berechnet auf die Zusammensetzung, mindestens eines filmbildenden Polymeren, aufbringt.

Bei beiden Zusammensetzungen handelt es sich um übliche, an sich bekannte Mittel; die Erfindung liegt in der oben definierten Kombination der Behandlungsschritte.

Die erfindungsgemäß im ersten Behandlungsschritt eingesetzte Zusammensetzung I enthält vorzugsweise etwa 0,5 bis 10, insbesondere etwa 1 bis 8 Gew.-% mindestens einer haarkonditionierenden langkettigen quaternären Ammoniumverbindung und/oder Aminverbindung.

Geeignete langkettige quaternäre Ammoniumverbindungen, die allein oder im Gemisch miteinander eingesetzt werden können, sind beispielsweise Cetyltrimethylammoniumchlorid, Dimethyldicetylammoniumchlorid, Trimethylcetylammoniumbromid, Stearyltrimethylammoniumchlorid, Dimethylstearylbenzylammoniumchlorid, Benzyltetradecylodimethylammoniumchlorid, Dimethyl- dihydriertes-Talgammoniumchlorid, Laurylpyridiniumchlorid, Lauryldimethylbenzylammoniumchlorid, Behenyltrimethylammoniumchlorid, Lauryltrimethylammoniumchlorid, Tris-(oligooxyethyl)alkylammoniumphosphat, Cetylpyridiniumchlorid, etc. Gut geeignet sind auch die in der EP-A 472 107 geoffenbarten quaternären Ammoniumsalze.
Im Prinzip sind alle quaternären Ammoniumverbindungen, wie sie im jeweils gültigen "CTFA International Cosmetic Ingredient Dictionary" unter dem Trivialnamen "Quaternium" aufgeführt sind, einsetzbar.

Besonders geeignete langkettige quaternäre Ammoniumverbindungen sind Esterquats der allgemeinen Formel (I) 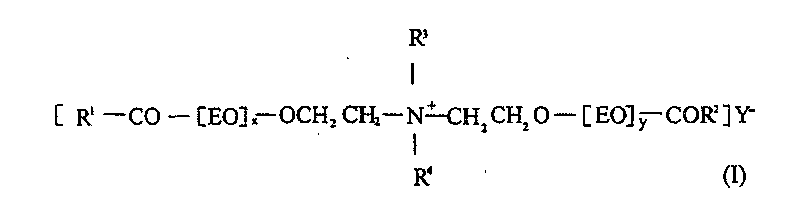 in der R¹ und R² für eine gegebenenfalls hydroxysubstituierte C₈-C₂₂-Alkyl- oder Alkenylgruppe, R³ und R⁴ für eine C₁-C₃-Alkylgruppe oder eine Gruppe -CH₂-CH₂-O-[EO]_{z}H sowie x, y und z für 0 bis 5 und Y⁻ für ein Anion stehen.

Eine besonders bevorzugte Verbindung der Formel I ist im Rahmen der Erfindung eine solche, in der die Reste R¹ und R² jeweils eine Oleylgruppe oder eine C₁₂-C₁₈-Alkylgruppe, der Rest R³ eine Methylgruppe und der Rest R⁴ eine Gruppe -CH₂-CH₂-O-[EO]_{z}-H bedeuten.
Das Anion Y⁻ ist vorzugsweise ein Halogenid wie Cl⁻ oder Br⁻, ein niederes Alkylsulfat, z.B. Methosulfat und Ethosulfat, oder ein Alkylphosphat, jedoch können selbstverständlich auch andere Reste eingesetzt werden.
Diese Verbindungen sind an sich bekannt und beispielsweise unter den Handelsnamen "Schercoquat®", "Dehyquart® F30" und "Tetranyl®" im Handel.
Der Einsatz dieser Verbindungen, sogenannter "Esterquats", in Haarpflegemitteln ist ebenfalls bereits bekannt und beispielsweise in der WO-A 93/10748, der WO-A 92/06899 und der WO-A 94/16677 beschrieben.
Geeignete haarkonditionierende langkettige Aminverbindungen sind vor allem C₁₀-C₂₄-Alkyl-, insbesondere C₁₂-C₁₈-Alkylamidoalkylendialkylamine der allgemeinen Formel (II) wie Stearamidopropyldimethyl- oder -diethylamin. 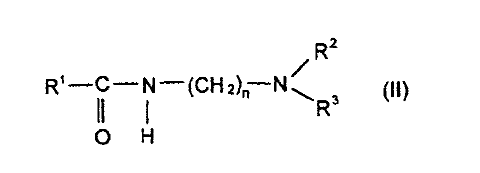 worin eine C₁₀-C₂₄-Alkylgruppe, R² und R³ eine C₁-C₃-Alkylgruppe und n 1 bis 5 bedeuten.

Die Zusammensetzung I kann natürlich weitere, an sich bekannte Wirk- und Zusatzstoffe enthalten.
Als solche können beispielsweise Fettsäuren, z.B. solche mit 10 bis 24, insbesondere 12 bis 22 Kohlenstoffatomen in einer Menge von etwa 0,5 bis 15 Gew.-%, insbesondere etwa 1 bis 10 Gew.-%, bezogen auf die Gesamtzusammensetzung I, eingesetzt werden. Besonders geeignet sind Behensäure und Stearinsäure; jedoch können auch andere Fettsäuren wie beispielsweise Myristinsäure, Palmitinsäure oder Ölsäure oder auch Gemische natürlicher oder synthetischer Fettsäuren wie Kokosfettsäure eingesetzt werden.

Das Haarbehandlungsmittel I kann als weiteren Bestandteil noch mindestens eine Verbindung, ausgewählt aus der Gruppe Harnstoff, 1-Methoxypropanol(-2), 1-Ethoxypropanol(-2), Diethylenglykolmonomethyl- oder -ethylether, Dipropylenglykolmonomethyl- oder -ethylether, Benzylalkohol, Benzyloxyethanol, Phenylethylalkohol, Phenoxyethanol und/oder Zimtalkohol, vorzugsweise in einer Menge von 0,5 bis 25, insbesondere 1 bis 20, vor allem 2,5 bis 15 Gew.-%, berechnet auf die Gesamtzusammensetzung des Mittels, enthalten. Bevorzugte Verbindungen aus dieser Gruppe sind Ethoxydiglykol und Benzyloxyethanol.

Die erfindungsgemäß verwendeten Haarbehandlungsmittel I können natürlich zusätzlich die in solchen Mitteln üblichen Bestandteile enthalten; es wird, zur Vermeidung von Wiederholungen, wiederum auf K. Schrader, "Grundlagen und Rezepturen der Kosmetika", 2. Aufl. (1989), S. 722-771, verwiesen.

Geeignete Zusatzstoffe sind beispielsweise auch synthetische oder natürliche haarkonditionierende Polymere, vorzugsweise in einer Menge von 0,1 bis 2,5, insbesondere 0,25 bis 1,5 Gew.-% der Gesamtzusammensetzung.
Besonders bevorzugt sind hierbei die unter der CTFA-Bezeichnung "Polyquaternium" bekannten kationischen (Co-)Polymeren, alleine oder auch im Gemisch mit nichtionischen, anionischen und/oder amphoteren Polymeren, beispielsweise solchen vom Typ "Amphomer^{R}".

Geeignete Fette und Öle, zu denen auch Wachse zählen, sind insbesondere natürliche Öle wie Avocadoöl, Cocosöl, Palmöl, Sesamöl, Erdnußöl, Spermöl, Sonnenblumenöl, Mandelöl, Pfirsichkernöl, Weizenkeimöl, Macadamianußöl, Nachtkerzenöl, Jojobaöl, Ricinusöl, oder auch Oliven- bzw. Sojaöl, Lanolin und dessen Derivate, ebenso Mineralöle wie Paraffinöl und Vaseline.
Synthetische Öle und Wachse sind beispielsweise Silikonöle, Polyethylengykole, etc. Weitere geeignete hydrophobe Komponenten sind insbesondere Fettalkohole, vorzugsweise solche mit etwa 8 bis 22 Kohlenstoffatomen im Molekül wie Myristyl-, Cetyl-, Stearylalkohol, Wachsalkohole und Fettsäureester wie Isopropylmyristat,-palmitat, -stearat und -isostearat, Oleyloleat, Isocetylstearat, Hexyllaurat, Dibutyladipat, Dioctyladipat, Myristylmyristat, Oleylerucat, Polyethylenglykol- und Polyglycerylfettsäureester wie PEG-7-glycerylcocoat, Cetylpalmitat, etc.

Diese hydrophoben Komponenten sind in der erfindungsgemäß verwendeten Zusammensetzung I vorzugsweise in einer Gesamtmenge von etwa 0,5 bis etwa 15, insbesondere etwa 1 bis 10, vor allem etwa 1,5 bis 7,5 Gew.-%, berechnet auf die Gesamtzusammensetzung, enthalten.

Ebenso können neben den oben erwähnten quaternären langkettigen Ammoniumverbindungen auch andere oberflächenaktive Stoffe, insbesondere amphotere bzw. zwitterionische und/oder nichtionische Tenside, deren einschlägige Verwendung natürlich an sich bekannt ist, eingesetzt werden.
Eine beispielhafte Zusammenfassung der Herstellung solcher Mittel findet sich ebenfalls in der bereits erwähnten Monographie von K. Schrader, S. 798 bis 818, insbesondere S. 804 ff.

Eine bevorzugte Tensidgruppe sind dabei die bekannten C₈-C₂₀-Alkylpolyglucoside, vorzugsweise solche mit einem Polymerisationsgrad von etwa 1,1 bis etwa 5, in einer bevorzugten Menge von etwa 0,5 bis etwa 20, insbesondere 1 bis etwa 10 Gew.-%, berechnet auf die Gesamtzusammensetzung.

Ein weiterer besonders geeigneter Zusatzstoff ist ein Ceramid der allgemeinen Formel (II) 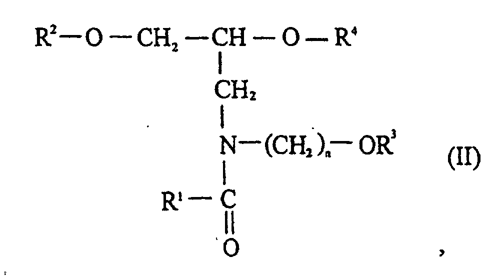 worin R¹ und R² gleiche oder verschiedene Alkyl- bzw. Alkenylreste mit 10 bis 22 Kohlenstoffatomen bedeuten, R³ für Wasserstoff oder eine Methyl-, Ethyl-, n-Propyl- oder Isopropylgruppe steht, R⁴ Wasserstoff, eine Hydroxymethyl-, Hydroxyethyl-, Dihydroxyethyl- oder Dihydroxypropylgruppe, und n eine ganze Zahl von 1 bis 6 bedeuten, insbesondere der Art, wie es aus der EP 227 994 A1 und der WO-A 96/37462 bekannt ist, jedoch sind auch andere Ceramide, beispielsweise die aus der WO-A 97/15724 oder der EP 647 617 B1 bekannten Ceramide, geeignet.

Die bevorzugten Gruppen R¹ und R² sind C₁₂-C₁₈-Alkylreste; n ist eine Zahl von 1 bis 3, R³ bedeutet vorzugsweise Wasserstoff oder einen Methylrest, und R⁴ Wasserstoff oder einen Dihydroxypropylrest.
Besonders bevorzugt sind Verbindungen, in denen R¹ einen C₁₂-C₂₄-Alkylrest, insbesondere eine C₁₃H₂₇-Alkylgruppe, R² einen C₁₄C₁₈-Alkylrest, insbesondere eine C₁₆H₃₃-Alkylgruppe, R³ einen Methylrest, R⁴ eine 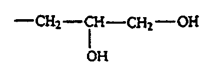 -Gruppe, und n 3 darstellen, oder eine Verbindung, wo R¹ für einen C₁₅-C₃₁-Alkylrest, R² für einen C₁₆H₃₃-Alkylrest, R³ und R⁴ für je ein Wasserstoffatom und n für 2 stehen.

Deren Menge in den erfindungsgemäß verwendeten Haarbehandlungsmitteln I liegt zweckmäßigerweise bei etwa 0,01 bis 10, vorzugsweise etwa 0,05 bis 7,5, insbesondere etwa 0,1n bis 5 Gew.-%, berechnet auf die Gesamtzusammensetzung.

Weitere Zusatzstoffe, deren Art und Menge natürlich von der Applikationsform des Mittels abhängig sind, sind Fette, Fettalkohole, Emulgatoren, pH-Regulatoren, Lösungs- und Verdünnungsmittel, Lösungsvermittler, Konservierungsmittel, Parfums, etc.

Die erfindungsgemäß als Zusammensetzung I verwendeten haarkonditionierenden Mittel liegen vorzugsweise als wäßrige oder wäßrig/alkoholische Lösung, wäßrige Emulsion, Mikroemulsion, Dispersion oder opakes oder transparentes Gel vor, und können auch als Aerosole konfektioniert werden. Solche Zusammensetzungen und ihre Herstellung sind dem Fachmann grundsätzlich bekannt und bedürfen daher keiner näheren Erläuterung.

Der pH-Wert der erfindungsgemäß verwendeten Haarbehandlungsmittel I ist nicht kritisch; er kann vorzugsweise bei 3 bis etwa 8, insbesondere zwischen 4 und 6,5, liegen.

Die in dem erfindungsgemäßen Verfahren als Zusammensetzung II zu verwendenden Mittel sind ebenfalls an sich bekannt.

Es kann sich dabei vorzugsweise um Lösungen, Emulsionen, Gele, Pumpsprays, Haarsprays und Schaumaerosole handeln.

Sie enthalten vorzugsweise etwa 0,5 bis 8, insbesondere etwa 1 bis 6 Gew.-% mindestens eines synthetischen oder natürlichen Polymeren.
Bevorzugt sind hierbei kationische Polymere, insbesondere dann, wenn es sich um wäßrige Zubereitungen wie Schaumaerosole handelt.
Geeignete haarkonditionierende kationische Polymere sind insbesondere die altbekannten quaternären Cellulosederivate des Typs "Polymer JR" und "Celquat" sowie quaternisierte Homo- und Copolymere des Dimethyldiallylammoniumchlorids, wie sie unter dem Handelsnamen "Merquat®" im Handel sind, quatemäre Vinylpyrrolidon-Copolymere, insbesondere mit Dialkylaminoalkyl(meth)acrylaten, wie sie unter dem Namen "Gafquat®" bekannt sind, Copolymerisate aus Vinylpyrrolidon und Vinylimidazoliniummethochlorid, die unter dem Handelsnamen "Luviquat®" angeboten werden, Polyamino-Polyamid-Derivate, beispielsweise Copolymere von Adipinsäure-Dimethylaminohydroxypropyldiethylentriamin, wie sie unter dem Namen "Cartaretine®F" vertrieben werden, sowie auch bisquaternäre langkettige Ammoniumverbindungen der in der US-PS 4 157 388 beschriebenen Harnstoff-Struktur, die unter dem Handelsnamen "Mirapol®A 15" im Handel sind.

Verwiesen wird in diesem Zusammenhang auch auf die in den DE-OSen 25 21 960, 28 11 010, 30 44 738 und 32 17 059 genannten kationaktiven Polymeren sowie die in der EP-A 337 354 auf den Seiten 3 bis 7 beschriebenen Produkte. Es könne auch Mischungen verschiedener kationischer Polymerer eingesetzt werden.

Zu den kationischen Polymeren zählen auch die in der EP-A 524 612 und der EP-A 640 643 beschriebenen Quaternisierungsprodukte aus Pfropfpolymerisaten von Organopolysiloxanen und Polyethyloxazolinen.

Grundsätzlich sind alle kationischen Polymeren, wie sie im CTFA International Cosmetic Ingredient Dictionary in der jeweils aktuellen Auflage unter der Bezeichnung "Polyquaternium" aufgeführt sind, geeignet.

Neben kationischen Polymeren ist auch der Einsatz von nichtionischen, amphoteren und/oder anionischen Filmbildnern möglich, insbesondere, wenn es sich um Haarsprays auf alkoholischer und/oder wäßrig alkoholischer Basis handelt.

Diese können nichtionische Polymere, vorzugsweise alkohol- und/oder wasserlösliche Vinylpyrrolidon-Polymere wie ein Vinylpyrrolidon-Homopolymerisat oder -Copolymerisat, insbesondere mit Vinylacetat, sein.
Geeignete Vinylpyrrolidon-Polymere sind z.B. die unter dem Handelsnamen "Luviskol®" bekannten Produkte, beispielsweise die Homopolymerisate "Luviskol® K 30, K 60 und K 90" sowie die wasser- bzw. alkohollöslichen Copolymerisate aus Vinylpyrrolidon und Vinylacetat, die unter dem Handelsnamen "Luviskol® VA 55 bzw. VA 64" von der BASF AG vertrieben werden.

Weitere geeignete nichtionische Polymere sind Vinylpyrrolidon/ Vinylacetat/Vinylpropionat-Copolymere wie "Luviskol®VAP 343", Vinylpyrrolidon/(Meth)Acrylsäureester-Copolymere sowie Chitosan-Derivate und Schellack.

Amphotere Polymere, die allein oder im Gemisch mit mindestens einem kationischen, nicht-ionischen und/oder anionischen Polymeren zum Einsatz gelangen, sind insbesondere Copolymerisate aus N-Octylacrylamid, (Meth)-Acrylsäure und tert.-Butylaminoethylmethacrylat vom Typ "Amphomer®"; Copolymerisate aus Methacryloylethylbetain und Alkylmethacrylaten vom Typ "Yukaformer®", z.B. das Butylmethacrylat-Copolymere "Yukaformer® Am 75"; Copolymerisate aus Carboxylgruppen und Sulfongruppen enthaltenden Monomeren, z.B. (Meth)Acrylsäure und Itaconsäure, mit basischen Gruppen, insbesondere Aminogruppen, enthaltenden Monomeren wie Mono- bzw. Dialkylaminoalkyl(meth)acrylaten bzw. Mono- bzw. Dialkylaminoalkyl(meth)acrylamiden; Copolymere aus N-Octylacrylamid, Methylmethacrylat, Hydroxypropylmethacrylat, N-tert.-Butylaminoethylmethacrylat und Acrylsäure sowie die aus der US-A 3,927,199 bekannten Copolymeren.

Falls erwünscht, können zusätzlich zu den oder anstelle der kationischen bzw. nichtionischen und/oder amphoteren Polymeren auch anionische Polymere verwendet werden.

Geeignete anionische Polymere sind Vinylalkylether-, insbesondere Methylvinylether/Maleinsäure-Copolymere, die durch Hydrolyse von Vinylether/Maleinsäureanhydrid-Copolymeren entstehen und unter der Handelsbezeichnung "Gantrez® AN oder ES" vertrieben werden. Diese Polymere können auch teilverestert sein, beispielsweise "Gantrez® ES 255", der Ethylester eines Ethylvinylethers/Maleinsäure-Copolymers, oder der Butyl- oder Isobutylester desselben.

Weitere geeignete anionische Polymere sind insbesondere Vinylacetat/Crotonsäure- oder Vinylacetat/Vinylneodecanoat/Crotonsäure-Copolymere des Typs "Resyn®", Natriumacrylat/Vinylalkohol-Copolymere des Typs "Hydagen® F", Natriumpolystyrolsulfonat, z.B. "Flexan® 130", Ethylacrylat/Acrylsäure/N-tert.-Butylacrylamid-Copolymere des Typs "Ultrahold®", Vinylpyrrolidon/Vinylacetat/Itaconsäure-Copolymere, Acrylsäure/Acrylamid-Copolymere bzw. Natriumsalze derselben vom Typ "Reten®"; etc.

Die eingesetzten Zusammensetzungen II können selbstverständlich alle üblichen, in solchen Mitteln zum Einsatz gelangenden Stoffe enthalten.

Als solche seien beispielhaft Komplexbildner, Lösungsmittel und Lösungsvermittler, Konservierungsmittel, pH-Regler, Weichmacher, Viskositätsregler wie anorganische Salze, soweit sie nicht ohnehin in den Tensid-Ausgangsmischungen enthalten sind, Duftstoffe, Verdickungsmittel, Feuchthaltemittel, pflanzliche und tierische Öle, etc. genannt.

Die möglichen Zusatzstoffe entsprechen im wesentlichen denjenigen, wie sie in der haarkonditionierenden Zusammensetzung I enthalten sein können.

Die Anwendung der Zusammensetzung I und II erfolgt in der Weise, daß auf vorzugsweise frisch gewaschenes Haar die Zusammensetzung I zur Einwirkung gebracht und dann, beispielsweise nach etwa 5 bis etwa 60 Minuten, ohne Ausspülen das Haar anschließend mit der Zusammensetzung II behandelt wird.

In den folgenden Beispielen wird das erfindungsgemäße Verfahren näher erläutert.

### Beispiel 1

| **Zusammensetzung I:** | |
|---|---|
| Behensäure | 1,0 (Gew.-%) |
| Avocadin^{R} | 0,5 |
| Verbindung der Formel I (R¹=R²=Oleyl; R³=CH₃; R⁴=CH₂-CH₂-OH; Y=CH₃SO₄⁻) | 1,0 |
| Steartrimoniumchlorid | 1,0 |
| 1,2-Propandiol | 5,0 |
| Cocoamidopropylbetain | 1,5 |
| C₁₂-C₂₄-Alkylpolyglucosid (P.D.∼1,5) | 2,5 |
| Harnstoff | 5,0 |
| Konservierungsmittel | 0,3 |
| Parfum | 0,3 |
| Wasser | ad 100,0 |

| **Zusammensetzung II:** | |
|---|---|
| Ethanol | 75,0 (Gew.-%) |
| Vinylacetat/Vinylpyrrolidon (80:20)-Copolymerisat | 7,0 |
| Quaternisiertes Vinylpyrrolidon/Dimethylamino-ethylmethacrylat-Copolymerisat | 0,5 |
| Dimethylsiloxan-Glycol-Copolymer (Dimethicone Copolyol) | 0,5 |
| Panthenol | 0,1 |
| Natriumisostearoyllactylat | 0,5 |
| Parfum | 0,1 |
| Wasser | ad 100,0 |

Diese Zusammensetzung wird aus einer bekannten mechanischen Pumpsprühvorrichtung appliziert.

In einem Doppelseitenversuch wurden an insgesamt 10 Probanden jeweils auf eine Kopfhälfte frisch gewaschenen Haares eine Zusammensetzung I und, nach zehnminütiger Einwirkung, ohne Ausspülen eine Zusammensetzung II aufgebracht.
Auf die andere Kopfhälfte von 5 Probanden wurde nur die Zusammensetzung I, auf die von weiteren 5 Probanden nur die Zusammensetzung II aufgebracht.
Nach 1 Stunde und 24 Stunden wurde die Eigenschaften anhand einer Punktbewertung von zwei unabhängigen Friseuren bewertet.

| **Ergebnis:** | | | |
|---|---|---|---|
| **Präferenzpunkte** | **Nach 1h/24h** | | |
| **Zusammensetzung:** | **I** | **II** | **I+II** |
| Eigenschaften: | | | |
| Glanz | 2/0 | 0/0 | 18/20 |
| Elastizität | 0/0 | 0/0 | 20/20 |
| Sprungkraft | 0/0 | 0/0 | 20/20 |
| Griff | 0/0 | 0/0 | 20/20 |
| Wiederfrisierbarkeit | 0/0 | 0/0 | 20/20 |

### Beispiel 2

| **Zusammensetzung I:** | |
|---|---|
| Stearamidopropyldimethylamin | 1,8 (Gew.-%) |
| Cetylalkohol | 4,0 |
| Ceteareth-20 | 0,5 |
| Glycerylstearat | 0,3 |
| Isopropylpalmitat | 0,3 |
| Ceramide (Sphingolipid E) | 0,1 |
| Parfum, Konservierungsmittel | q.s. |
| Wasser | ad 100,0 |

| **Zusammensetzung II:** | |
|---|---|
| Cetrimoniumchlorid | 0,5 (Gew.-%) |
| Hydroxyethylcellulose | 0,1 |
| PEG-40-hydriertes Ricinusöl | 0,2 |
| Laureth-4 | 0,2 |
| Polyquaternium-16 | 2,0 |
| Vinylpyrrolidon/Vinylacetat-Copolymerisat (Luviskol^{R}VA64) | 1,0 |
| Ethanol | 10,0 |
| Konservierungsmittel, Parfum | q.s. |
| Wasser | ad 100,0, |

abgefüllt mit 10 Gew.-% Propan/Butan-Treibmittelgemisch.

Auf frisch gewaschenes Haar wurden zunächst die Zusammensetzung I und, nach etwa fünfzehn Minuten, ohne Ausspülen, die Zusammensetzung II aufgebracht. Es wurde ein glänzendes, elastisches und flexibles Haar mit weichem Griff und vollem Volumen erhalten, dessen Frisur nach 24 Stunden gut wiederherstellbar war.

### Beispiel 3

| **Zusammensetzung I:** | |
|---|---|
| Dioleoylethylhydroxyethylmethosulfat | 1,0 (Gew.-%) |
| Dimethicone Copolyol Bienenwachs | 1,0 |
| Cetylstearylalkohol | 1,0 |
| C₈-C₁₀-Alkylpolyglucosid (P.D.∼1,4) | 1,5 |
| 1,2-Propandiol | 2,0 |
| Harnstoff | 4,0 |
| Hydroxyethylcellulose | 0,8 |
| Saccharose | 0,5 |
| Behentrimoniumchlorid | 0,4 |
| Grüner Tee-Extrakt | 0,3 |
| Isostearylglycerylether | 0,2 |
| Parfum | 0,3 |
| Konservierungsmittel | 0,3 |
| Farbstoffe, UV-Absorber | q.s. |
| Wasser | ad 100,0 |

| **Zusammensetzung II:** | |
|---|---|
| Organopolysiloxan A-1 (nach "Synthese-Beispiel 1" der EP-A 640 643) | 1,0 (Gew.-%) |
| N-Octylacrylamid/tert.-Butylaminoethylmethacrylat/ Acrylsäure-Copolymerisat (Amphomer®) | 5,0 |
| 2-Amino-2-methyl-1-propanol | 1,0 |
| PEG-40-hydriertes Ricinusöl | 0,1 |
| Parfum | 0,2 |
| Dimethylether | 33,0 |
| Wasser | ad 100,0 |

Zunächst wurde, wie in den Beispielen 1 und 2 beschrieben, die Zusammensetzung I auf frisch gewaschenes Haar aufgebracht und nach 30 Minuten dieses Haar ohne Ausspülen mit dem Haarspray der Zusammensetzung II behandelt.
Das Haar war nach 36 Stunden noch leicht wiederfrisierbar, wies Sprungkraft, Elastizität, einen weichen vollen Griff und angenehmen Glanz auf.

### Beispiel 4

| **Zusammensetzung I:** | |
|---|---|
| Behensäure | 2,0 (Gew.-%) |
| Stearinsäure | 1,0 |
| N-(3-Hexadecyloxy-2-hydroxypropyl)-N-2- | |
| hydroxyethyldecanamid (Ceramid) | 0,5 |
| Stearamidopropyldimethylamin | 3,0 |
| Steartrimoniumchlorid | 1,0 |
| 1,2-Propandiol | 2,5 |
| Harnstoff | 5,0 |
| C₈-C₂₀-Alkylpolyglucosid (P.D.∼ 1,4) | 4,5 |
| Parfum | 0,3 |
| Konservierungsmittel | 0,3 |
| Wasser | ad 100,0 |

| **Zusammensetzung II:** | |
|---|---|
| Organopolysiloxan A-1 (nach "Synthese-Beispiel 1" der EP-A 640 643) | 3,0 (Gew.-%) |
| Methacrylsäure/Ethylacrylat/tert.-Butylacrylat/ Copolymerisat (Luvimer®36D) | 5,0 |
| 2-Amino-2-methyl-1-propanol | 1,1 |
| Ceteareth-25 | 0,3 |
| Laureth-4 | 0,2 |
| PEG-40-hydriertes Ricinusöl | 0,1 |
| Parfum | 0,2 |
| Propan/Butan-Treibmittelgemisch | 10,0 |
| Wasser | ad 100,0 |

Auf frisch gewaschenes Haar wurde zunächst eine Emulsion der Zusammensetzung I und dann, nach etwa fünfminütiger Einwirkung, sofort ein Aerosolschaum der Zusammensetzung II aufgebracht.

Das Haar zeigte nach 4, 24 und sogar 48 Stunden einen dezenten Glanz, Fülle, weichen und elastischen Griff und Sprungkraft; die ursprüngliche Löckchenfrisur ließ sich auch nach 48 Stunden wiederherstellen.

### Beispiel 5

| **Zusammensetzung I:** | |
|---|---|
| | |
| Cetylstearylalkohol | 5,00 (Gew.-%) |
| Dicetyldimoniumchlorid | 1,60 |
| Klettenwurzelölextrakt | 1,00 |
| Hydroxypropyl-Guar-hydroxypropyltrimonium- | 0,40 |
| chlorid | |
| Isopropylalkohol | 1,50 |
| Isopropylmyristat | 1,20 |
| Panthenol | 0,50 |
| Parfum | 0,40 |
| Milchsäure | 0,55 |
| Citronensäure | 0,05 |
| Pyrrolidoncarbonsäure | 0,02 |
| Weinsäure | 0,02 |
| Apfelsäure | 0,01 |
| Konservierungsmittel | q.s. |
| Wasser | ad 100,00 |

| **Zusammensetzung 11:** | |
|---|---|
| | |
| Vinylacetat/Crotonsäure-Copolymerisat (Aristoflex®A) | 3,0 (Gew.-%) |
| Polyquaternium-11 | 1,0 |
| Polyquaternium-14 | 0,5 |
| Laureth-12 | 0,5 |
| Ethanol | 8,0 |
| Parfum, Konservierungsmittel | q.s. |
| Wasser | ad 100,0 |

mit Propan/Butan-Treibmittelgemisch im Verhältnis 10:1 abgefüllt.

Auf frisch gewaschenes Haar wurde zunächst die Zusammensetzung I und anschließend, nach etwa 30-minütiger Einwirkung, ohne zwischenzeitliches Ausspülen die Zusammensetzung II aufgebracht.
Nach 2, 8 und 24 Stunden wurde das Haar begutachtet und gegenüber den Einzelbehandlungen ein besserer Glanz, verbesserte Elastizität und Sprungkraft, weicherer, vollerer Griff und Volumen sowie eine deutlich erhöhte Wiederfrisierbarkeit konstatiert.

## Patentansprüche

1. Verfahren zum Behandeln von frisch gewaschenem Haar, wobei auf das Haar zunächst eine Zusammensetzung I, enthaltend 0,25 bis 15 Gew.-%, berechnet auf die Zusammensetzung I, mindestens einer haarkonditionierenden langkettigen quaternären Ammoniumverbindung und/oder Aminverbindung aufgebracht und anschließend, ohne zwischenzeitliches Ausspülen, mit einer Zusammensetzung II, enthaltend 0,25 bis 10 Gew.-%, berechnet auf die Zusammensetzung II, mindestens eines filmbildenden Polymeren, behandelt wird.

2. Verfahren nach Anspruch 1, **dadurch gekennzeichnet, daß** die Zusammensetzung II mindestens ein kationisches Polymeres enthält.

3. Verfahren nach Ansprüch 1 und/oder 2, **dadurch gekennzeichnet, daß** die Zusammensetzung II als Schaumaerosol vorliegt.

4. Verfahren nach einem oder mehreren der Ansprüche 1 bis 3, **dadurch gekennzeichnet, daß** die Zusammensetzung I etwa 0,5 bis 10 Gew.-% mindestens einer langkettigen quaternären Ammoniumverbindung enthält.

5. Verfahren nach einem oder mehreren der Ansprüche 1 bis 3, **dadurch gekennzeichnet, daß** die Zusammensetzung I etwa 0,5 bis 10 Gew.-% mindestens eines langkettigen Amins der allgemeinen Formel 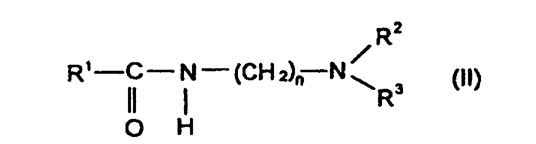 worin R¹ eine C₁₀-C₂₄-Alkylgruppe, R² und R³ eine C₁-C₃-Alkylgruppe und n 1 bis 5 bedeuten, enthält.

6. Verfahren nach Anspruch 5, **dadurch gekennzeichnet, daß** R¹ eine C₁₂-C₁₈-Alkylgruppe, R² und R³ eine Methyl- oder Ethylgruppe und n 2 oder 3 bedeuten.

## Claims

1. Process for the treatment of freshly shampooed hair, whereby first a Composition I, containing 0.25 % to 15 % by weight, calculated to the total Composition I, of at least one hair-conditioning long-chain quaternary ammonium compound and/or amine compound is applied onto the hair, and subsequently, without interim rinsing, the hair is treated with a Composition II, containing 0.25 % to 10 % by weight, calculated to the total Composition II, of at least one film-forming polymer.

2. Process according to claim 1, wherein Composition II contains at least one cationic polymer.

3. Process according to claim 1 and/or 2, wherein the Composition II is present as foam aerosol.

4. Process according to one or more of claims 1 to 3, wherein the Composition I contains about 0.5 % to 10 % by weight of at least one long-chain quaternary ammonium compound.

5. Process according to one or more of claims 1 to 3, wherein the Composition I contains about 0.5 % to 10 % by weight of at least one long-chain amine of the general formula 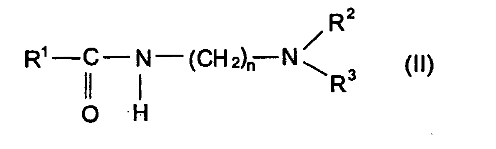 wherein R¹ is a C₁₀-C₂₄-alkyl group, R² and R³ are a C₁-C₃-alkyl group and n is 1 to 5.

6. Process according to claim 5, wherein R¹ is a C₁₂-C₁₈-alkyl group, R² and R³ are a methyl or ethyl group, and n is 2 or 3.

## Revendications

1. Procédé destiné à traiter des cheveux fraîchement lavés, une composition I, contenant de 0,25 à 15 % du poids de la composition I d'au moins un composé quaternaire d'ammonium à chaîne longue et conditionneur du cheveu et/ou d'un composé amine étant appliquée puis, sans rinçage intermédiaire un traitement étant effectué à l'aide d'une composition II, contenant de 0,25 à 10 % du poids de la composition II, d'au moins un polymère filmogène.

2. Procédé selon la revendication 1,
**caractérisé en ce que**
le composé II contient au moins un polymère cationique.

3. Procédé selon l'une quelconque des revendications 1 ou 2,
**caractérisé en ce que** la composition II est présente sous forme d'aérosol mousse.

4. Procédé selon l'une quelconque ou plusieurs des revendications 1 à 3,
**caractérisé en ce que**
la composition I contient environ de 0,5 à 10 % en poids d'au moins un composé ammonium quaternaire à chaîne longue.

5. Procédé selon l'une quelconque ou plusieurs des revendications 1 à 3,
**caractérisé en ce que**
la composition I contient environ de 0,5 à 10 % en poids d'au moins un amine à chaîne longue, de la formule générale 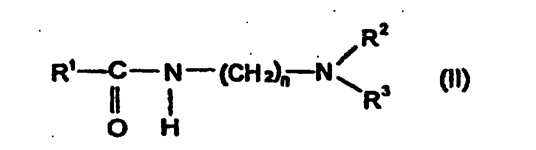 R¹ signifiant un groupe alkyle en C₁₀ à C₂₄, R² et R³ signifiant un groupe alkyle en C₁ à C₃ et n signifiant de 1 à 5.

6. Procédé selon la revendication 5,
**caractérisé en ce que**
R₁ signifie un groupe alkyle en C ₁₂- à C ₁₈₋, R² et R³ signifiant un groupe méthyle ou un groupe éthyle et n signifiant 2 ou 3.
